# EUROPÄISCHE PATENTANMELDUNG

(11) **EP 2 742 959 A2**
(43) Veröffentlichungstag der Anmeldung: **18.06.2014**
(21) Anmeldenummer: 13005587.4
(22) Anmeldetag: 02.12.2013
(51) Int. Cl.: A61M 5/00, A61M 5/24, A61M 5/32

(54) **Vorrichtung zur Aufbewahrung Lagerung von flüssigen Medien, insbesondere Arzneimitteln**

(30) Priorität: 13.12.2012 DE 102012024371
(71) Anmelder: Schott AG, 55122 Mainz (DE)
(72) Erfinder: Langsdorf, Andreas, 55218 Ingelheim (DE); Henze, Inka, 55268 Nieder-Olm (DE); Beier, Wolfram, 55270 Essenheim (DE); Ohlinger, Axel, 65205 Wiesbaden (DE)
(74) Vertreter: Sawodny, Michael-Wolfgang

(57) **Zusammenfassung**

Die Erfindung betrifft eine Vorrichtung zur Aufbewahrung und Lagerung von flüssigen Medien, insbesondere Arzneimitteln mit einem Spritzenkörper (10), umfassend einen Aufbewahrungs- und/oder Lagerbehälter (20), insbesondere aus einem Glasmaterial für flüssige Medien, insbesondere Arzneimittel, mit einem Verschluss (30), insbesondere aus einem gummielastischen Werkstoff sowie wenigstens einer Nadel (200).

Die Erfindung ist dadurch gekennzeichnet, dass der Aufbewahrungs- und/oder Lagerbehälter als Fläschchen mit einem festen Boden und die Vorrichtung eine Schiebe-, insbesondere eine Drückeinrichtung umfasst zur Verschiebung des Aufbewahrungs- und/oder Lagerbehälters im Wesentlichen in axialer Richtung in dem Spritzenkörper, wobei die Schiebe-, insbesondere Drückeinrichtung auf den festen Boden wirkt.

## Beschreibung

Die Erfindung betrifft eine Vorrichtung zur Aufbewahrung und Lagerung von flüssigen Medien, insbesondere Arzneimitteln sowie ein Verfahren zum Ausbringen von flüssigen Medien, insbesondere Arzneimitteln aus einer Vorrichtung zur Aufbewahrung und/oder Lagerung von flüssigen Medien, insbesondere Arzneimitteln.

Spritzen, insbesondere Glasspritzen, die zur Aufbewahrung und Lagerung von flüssigen Medien dienen, sind aus dem Stand der Technik bekannt geworden. So beschreibt die EP 1 600 190 A1 oder die US 6,190,364 einen Spritzenkörper zum Aufbewahren und/oder Lagern eines in den Spritzenkörper eingebrachten flüssigen Mediums, beispielsweise eines Arzneimittels, wobei der zur Lagerung bzw. Aufbewahrung des Fluids dienende Spritzenkörper mit einem Spritzenverschluss abdichtend an einem distalen Ende verschlossen ist. Zum Injizieren der Spritzenflüssigkeit wird bei der EP 1 600 190 A1 oder der US 6,190,364 eine Verschlusskappe auf einen Befestigungsring geschraubt und eine Spritzennadel an den Befestigungsring und damit an dem Spritzenkörper so befestigt, dass eine sich durch die Spritzennadel erstreckende Nadelöffnung in Strömungsverbindung mit der distalen Öffnung des Spritzenkörpers steht. Nachteilig an den Behältern zur Lagerung bzw. Aufbewahrung von flüssigen Arzneimitteln gemäß der EP 1 600 190 A1 bzw. US 6,190,364 war, dass, nachdem die Spritzenflüssigkeit bzw. das Fluid durch die Nadel aus dem Spritzenkörper verbracht war, die Nadel am Spritzenkörper überstand und die benutzten Spritzenkörper mit der herausragenden Nadel ein Verletzungsrisiko darstellten.

Nadelspritzen, deren Nadel nach Verabreichung noch überstehen bzw. herausragen, können zu Nadelstichen führen, was zu der Nachbehandlung besonders von Klinikpersonal führt.

Aus der US 6,015,438 ist für eine Kunststoffspritze, die nicht als Lager- und Aufbewahrungsbehälter für ein Arzneimittel dienen kann, eine Vorrichtung bekannt geworden, bei der eine Spritzennadel nach Gebrauch in die Spritze eingezogen werden kann.

Nachteilig an dem System gemäß der US 6,015,438 war, dass das in der US 6,015,438 angegebene System vor dem Verabreichen befüllt werden musste, da das Kunststoffmaterial der Spritze gemäß der US 6,015,438 nicht für eine dauerhafte Lagerung von Arzneimitteln geeignet war

Außerdem kommen in dem System gemäß der US 6,015,438 viele unterschiedliche Komponenten zum Einsatz, die direkten Kontakt zum Arzneimittel haben und eine Zulassung benötigen. Darüber hinaus ist die Herstellung vieler kleiner Bauteile aufwendig. Weiterhin nachteilig ist es, dass die Nadel in die Kolbenstange zurückgezogen werden muss. Dies bedingt eine hohle Kolbenstange.

Die DE 697 28 062 beschreibt eine Möglichkeit, eine Nadel derart zu halten, dass sie erst nach Abzug der Kappe ein Septum durchstößt und damit bis zum Gebrauch keinen Kontakt zum Arzneimittel hat. Nachteilig ist auch hier, dass die Kolbenstange besonders ausgestaltet sein muss, um die Nadel nach Gebrauch in der Spritze aufzunehmen.

Aus der WO 2010/100244 ist eine Vorrichtung zum Verabreichen eines Arzneimittels bekannt geworden, bei dem zum Ausbringen des Arzneimittels zunächst eine Versiegelung, bevorzugt von einer Nadel, durchstoßen wird. Wenn das Arzneimittel vollständig ausgebracht ist, kann die Nadel durch den Kolben, mit dem das Arzneimittel ausgebracht wurde, wieder in den Spritzenkörper eingezogen werden. Allerdings erfolgt dies nicht automatisch nach dem Ausbringen, sondern die Nadel muss aktiv in den Kolben zurückgezogen werden. Ein weiteres Problem bei der WO 2010/100244 war, dass eine Wiederverwertung nicht sicher ausgeschlossen werden konnte.

Die WO 2012/000838 und die WO 2012/000832 beschreiben eine Spritze, mit der ein Arzneimittel über eine Nadel abgegeben werden kann. Auch bei der WO 2012/000838 und der WO 2012/000832 wird das Arzneimittel aus dem Aufbewahrungsbehälter mittels eines Kolbens ausgedrückt. Nach Ausdrücken des Arzneimittels wird dann die Nadel mit dem gesamten Aufnahmebehälter wieder eingezogen. Nachteilig an diesem System war zum einen der relativ große Bauraum, zum anderen, dass eine Mehrfachverwendung nicht sicher ausgeschlossen werden konnte und die große Anzahl an einzelnen Bauelementen.

Aus der WO 2011/106870 ist eine Vorrichtung bekannt geworden, bei der aus einer mit einem Stempel verschlossenen Karpule ein Septum ausgebracht wird. Nachteilig an der WO 2011/106870 ist, dass der Stempel ein separates Bauteil ist.

Die US 2009/0259195 A1 zeigt eine Spritze mit einem Behälter, der einen beweglichen Dichtring aufweist. Die Verschiebung des beweglichen Dichtringes hat den Nachteil, dass eine Vielzahl von Komponenten sterilisiert werden müssen.

Die US 2009/0259195 A1 beschreibt des Weiteren eine Drückeinrichtung, die sich innerhalb des hohlen Aufbewahrungsbehälters bewegt.

Aus der US 2,549,417 ist ein Behälter mit einem Verschluss und einem festen Boden bekannt geworden, der Verwendung in einer Spritze verwendet werden kann.

Die US 6,015,438 zeigt eine Spritze mit einer Nadelrückzieheinrichtung.

Aufgabe der Erfindung ist es, die aus dem Stand der Technik bekannten Nachteile zu überwinden und insbesondere eine Vorrichtung zur Aufbewahrung und Lagerung von flüssigen Medien, insbesondere Arzneimitteln, mit einem Spritzenkörper anzugeben, der zum einen eine Aufbewahrung ermöglicht, zum anderen das Verletzungsrisiko wie beispielsweise bei einem System gemäß der EP 1 600 190 A1 vermeidet. Weiterhin ist es Aufgabe der Erfindung, möglichst wenige Kontaktpartner für die Arzneimittel anzubieten.

Erfindungsgemäß wird diese Aufgabe dadurch gelöst, dass eine Vorrichtung zur Aufbewahrung und Lagerung von flüssigen Medien, insbesondere Arzneimitteln, angegeben wird, mit einem Spritzenkörper, umfassend einen Aufbewahrungs- und/oder Lagerbehältermit einem festen Boden, insbesondere in Form eines Fläschchens insbesondere aus einem Glasmaterial, einem Cyclooelefincopolymeren (COC) oder Cyclooelefinpolymer ( COP) für flüssige Medien, insbesondere Arzneimittel, mit einem Verschluss, insbesondere aus einem gummielastischen Werkstoff sowie wenigstens einer Nadel und einer Nadelrückzieheinrichtung.

Unter einem Fläschchen wird in vorliegender Anmeldung ein wenigstens einseitig offener Körper verstanden. So ist beispielsweise als Aufbewahrungs- und/oder Lagerbehälter für die Erfindung ein einseitig offener Körper - also ein Fläschchen - nutzbar mit einem festen Boden. Ein Fläschchen mit zwei offenen Enden wird auch als Karpule bezeichnet. Demgegenüber ist eine Ampulle ein vollkommen verschlossener Körper. So hat beispielsweise eine Glasampulle zwei verschmolzene Enden. Während eine Glasampulle bei der Erfindung keine Verwendung finden kann, wäre das für eine Karpule möglich, wenn eines ihrer offenen Enden, z. B. an der Unterseite der Karpule verschlossen wird. Eine besonders vorteilhafte Form eines Aufbewahrungs- und/oder Lagerbehälters, der in vorliegender Erfindung Verwendung finden kann, ist ein Körper in Rohrform mit einem festen Ende.

Erfindungsgemäß umfasst die Vorrichtung eine Schiebe-, insbesondere eine Drückeinrichtung zur Verschiebung des Aufbewahrungs- und/oder Lagerbehälters im Wesentlichen in axialer Richtung in dem Spritzenkörper. Durch die Verschiebung des gesamten Aufbewahrungs- und/oder Lagerbehälters wird das Arzneimittel ausgebracht, ohne dass ein Kolben in dem Aufbewahrungs- und/oder Lagerbehälter bewegt werden muss, vielmehr wirkt die Drückeinrichtung auf den Boden des Aufbewahrungs- und/oder Lagerbehälters. Durch diese Maßnahme besteht insbesondere kein Kontakt des Kolbens mehr zu dem Arzneimittel, wie beispielsweise im Fall der WO 2012/000838, der WO 2012/000832 oder der WO 2011/106870. Hierdurch kann die Anzahl der Komponenten, die in Kontakt mit dem Arzneimittel stehen, ganz erheblich reduziert werden. Im Gegensatz zum Stand der Technik stehen nur noch zwei Materialien in direktem Medikamentenkontakt, so dass die erfindungsgemäße Vorrichtung zum Aufbewahren und Lagern von Arzneimitteln leichter sterilisierbar ist. Des Weiteren wird durch die Ausgestaltung mit einem festen Boden die Anzahl der Öffnungen des Lagerbehälters reduziert, so dass die Anzahl der Dichtungen, insbesondere die zu bewegenden Bauteile wie Kolben, verringert wird. Derartige Dichtungen sind immer kritisch in Bezug auf Sterilität und Kontakt mit Medikamenten.

Als Materialien für den Lager- und Aufbewahrungsbehälter werden bevorzugt aus einem Material verwendet, das eine Langzeitlagerung von Arzneimitteln von wenigstens drei Jahren ermöglicht. Als besonders bevorzugte Materialien sind sämtliche Glas- und Glaskeramikmaterialien geeignet, aber auch Polymermaterialien, die ausreichend beständig sind, wie beispielsweise Cyclooelefincopolymere (COC) oder Cyclooelefinpolymere (COP).

Zum Ausdrücken des im Lager- und/oder Aufbewahrungsbehälter befindlichen Arzneimittels umfasst die erfindungsgemäße Vorrichtung eine Schiebe-, insbesondere eine Drückeinrichtung zur Verschiebung des Aufbewahrungs- und/oder Lagerbehälters, im Wesentlichen in axialer Richtung in dem Spritzenkörper.

Die Schiebe- und/oder Drückeinrichtung wirkt dabei auf den Boden des Aufbewahrungs- und/oder Lagerbehälters. Nach Durchstoßen eines Stopfens durch die Nadel, der die Öffnung des Aufbewahrungs- und/oder Lagerbehälters verschließt, wird durch Verschieben des Lagerbehälters in axialer Richtung das Arzneimittel ausgebracht.

Bevorzugt weist die Druckeinrichtung wenigstens einen Vorsprung auf. Beim Ausbringen des Arzneimittels mit der Drückeinrichtung gleitet der Vorsprung innerhalb des Spritzenkörpers, wodurch die Enden der Wände der bevorzugt als Hohlzylinder mit wenigstens einer Öffnung ausgebildeten Druckvorrichtung auf den Behälterboden bzw. den festen Boden wirken können.

In einer vorteilhaften Ausführungsform ist somit der Vorsprung derart ausgebildet, dass die Drückeinrichtung zum Verschieben des Aufbewahrungs- und/oder Lagerbehälters zum Ausbringen des flüssigen Mediums mit dem Aufbewahrungs- und/oder Lagerbehälter zur Anlage kommt.

In einer weitergebildeten Ausführungsform ist der Vorsprung derart ausgebildet, dass nach Ausbringen des flüssigen Mediums aus dem Aufbewahrungs- und/oder Lagerbehälter der Aufbewahrungs- und/oder Lagerbehälter von der Drückeinrichtung, die insbesondere als Hohlzylinder ausgebildet ist, aufgenommen werden kann. Hierzu verrastet der Vorsprung in einer Aussparung in der Wand des Spritzenkörpers, wodurch die Öffnung des Hohlzylinders freigegeben wird, so dass der Aufbewahrungs- und/oder Lagerbehälter vom Inneren des Hohlzylinders nach Ausbringen des Arzneimittels aufgenommen werden kann.

Bevorzugt weist der Spritzenkörper des Weiteren ein vorderes Ende auf, wobei die Nadelrückzieheinrichtung in dem vorderen Ende, bevorzugt durch eine Halteeinrichtung, gehalten wird. Um die Nadelrückzieheinrichtung freizugeben, ist bevorzugt vorgesehen, dass die Vorrichtung eine Auslösevorrichtung für die Nadelrückzieheinrichtung umfasst, beispielsweise in Form eines Federelements oder ganz besonders bevorzugt in Form einer Kralle.

In einer ersten Ausgestaltung der Erfindung umfasst die Nadelrückzieheinrichtung eine Nadelhalteeinrichtung und eine Druckfeder.

Der Aufbewahrungs- und/oder Lagerbehälter der Vorrichtung zur Aufbewahrung und/oder Lagerung von flüssigen Medien, insbesondere Arzneimitteln, ist bevorzugt aus einem Glasmaterial hergestellt und in dem Spritzenkörper bevorzugt axial verschiebbar angeordnet. Es handelt sich dann um Glasfläschchen mit einem festen Boden. Besonders bevorzugte Glasmaterialien sind Glasmaterialien, die für die Lagerung im Packmittel zugelassen sind. Ein derartiges Glasmaterial ist beispielsweise das unter dem Markennamen FIOLAX vertriebene Glas der Schott AG Mainz. Das Glas FIOLAX klar umfasst als Hauptbestandteile 75 Gew.-% SiO₂, 10,5 Gew.-% B₂O₃, 5 Gew.-% Al₂O₃, 7 Gew.- % Na₂O und 1,5 Gew.-% CaO. Es weist einen mittleren linearen thermischen Ausdehnungskoeffizienten α (20°C, 300°C) von 4,9·10⁻⁶K⁻¹, eine Transformationstemperatur Tg von 565°C und eine Dichte p bei 25°C von 2,34g cm⁻³ auf.

Dadurch, dass der Aufbewahrungs- und/oder Lagerbehälter in dem Spritzenkörper im Wesentlichen in axialer Richtung verschiebbar ausgebildet ist, kann durch Verschieben des Aufbewahrungs- und/oder Lagerbehälters beispielsweise mittels einer Schiebe- oder Drückeinrichtung, bevorzugt in Form eines Spritzenkolbens, der Lager- und/oder Aufbewahrungsbehälter verschoben und in dem Lager- oder Aufbewahrungsbehälter vorhandenes flüssiges Medium über eine Nadel ausgebracht werden. Nachdem die Flüssigkeit vollständig aus dem Lager-und/oder Aufbewahrungsbehälter ausgebracht wurde, kann die Nadelrückzieheinrichtung ausgelöst werden und mit Hilfe der Nadelrückzieheinrichtung die Nadel vollständig in den Spritzenkörper zurückgezogen werden.

Neben der Vorrichtung gibt die Erfindung auch ein Verfahren zum Ausbringen von flüssigen Medien, insbesondere Arzneimitteln, aus einer Vorrichtung zur Aufbewahrung und/oder Lagerung an. Bei einem erfindungsgemäßen Verfahren wird zunächst der Verschluss des Aufbewahrungs- und/oder Lagerbehälters durch einen Einstich einer Nadel geöffnet, die bevorzugt in einer Nadelhalteeinrichtung gehalten wird. Aus dem geöffneten Aufbewahrungs- und/oder Lagerbehälter strömt dann das flüssige Medium, insbesondere das Arzneimittel, aus, wobei durch ein axiales Verschieben des Aufbewahrungs- und/oder Lagerbehälters in dem Spritzenkörper das gesamte flüssige Medium durch die Nadel ausgebracht werden kann.

Nach weitgehend vollständigem Ausbringen des flüssigen Mediums, insbesondere Arzneimittels, wird dann beispielsweise mittels einer Auslöseeinrichtung die Nadelrückzieheinrichtung freigegeben derart, dass die Nadel in den Spritzenkörper weitgehend vollständig eingezogen wird. Besonders bevorzugt ist es, wenn die Auslöseeinrichtung dazu dient, eine Halteeinrichtung einer Nadelhalteeinrichtung zu öffnen, derart, dass bevorzugt eine Druckfeder sich entspannt und dadurch die Nadelhalteeinrichtung zusammen mit der Nadel in das Innere des Spritzenkörpers gezogen wird. Hierbei ist es besonders bevorzugt, wenn die Druckeinrichtung zur Verschiebung des Aufbewahrungs- und/oder Vorratsbehälters derart ausgebildet ist, dass die Drückeinrichtung einen Hohlraum umfasst, der die zurückgezogene Nadel sowie den mit der Nadel in axialer Richtung zurückgedrückten Vorrats- und/oder Aufbewahrungsbehälter aufnimmt. Bevorzugt ist die Drückeinrichtung ein Hohlzylinder.

Besonders bevorzugt ist es, wenn die Nadelhalteeinrichtung im vorderen Ende des Spritzenkörpers gehalten wird, beispielsweise durch einen Form- und/oder Reibschluss und der Form- und/oder Reibschluss der Halteeinrichtung mit dem vorderen Ende des Spritzenkörpers durch die Auslöseeinrichtung aufgehoben und dadurch die Nadelrückzieheinrichtung freigegeben wird. Durch die Freigabe der Nadelrückzieheinrichtung entspannt sich die Druckfeder und zieht die Nadelrückzieheinrichtung zusammen mit der Nadel in das Innere des Spritzenkörpers zurück.

Um das flüssige Medium weitgehend vollständig aus dem Lager- und/oder Aufbewahrungsbehälter auszubringen, ist vorgesehen, dass der Verschluss des Lager- und/oder Aufbewahrungsbehälters mit einem gummielastischen Werkstoff verschlossen wird. Dieser Werkstoff lässt sich bevorzugt derart komprimieren, dass das flüssige Medium, das in dem Lager- und/oder Aufbewahrungsbehälter gelagert wird und weitgehend vollständig ausgebracht werden kann.

Die Erfindung soll nachfolgend anhand der Figuren eingehend beschrieben werden, ohne hierauf beschränkt zu sein.

Es zeigen:
- Figur 1: eine erfindungsgemäße Ausführungsform eines Spritzenkörpers mit den Einzelteilen im Aufriss;
- Figur 2: den Spritzenkörper nach Einbringen der Nadel und vor Ausbringen des flüssigen Mediums;
- Figur 3: den Spritzenkörper nach Einbringen der Nadel in den Lager- und/oder Aufbewahrungsbehälter und nach Ausdrücken eines Teils des flüssigen Mediums;
- Figur 4: den Spritzenkörper nach Auspressen des gesamten flüssigen Mediums, aber vor Betätigen der Auslöseeinrichtung;
- Figur 5: den Spritzenkörper nach Betätigen der Auslöseeinrichtung und der Nadelrückzieheinrichtung;
- Figur 6: detaillierte Darstellung der Auslöseeinrichtung vor Auslösen;
- Figur 7: detaillierte Darstellung der Auslöseeinrichtung beim Auslösen;
- Figur 8: sterile Einheit des Spritzenkörpers.

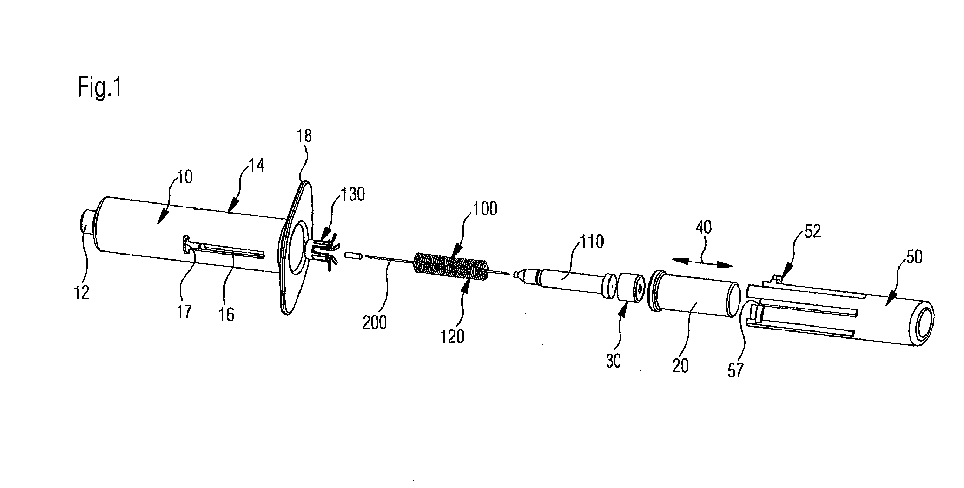

In Fig. 1 sind die Einzelteile der erfindungsgemäßen Vorrichtung zur Aufbewahrung und Lagerung von flüssigen Medien, insbesondere Arzneimitteln, im auseinander gebauten Zustand gezeigt. In einer besonders vorteilhaften, aber nicht hierauf beschränkten Ausführungsform umfasst die Vorrichtung zur Aufbewahrung und Lagerung von flüssigen Medien einen Spritzenkörper 10. Im Spritzenkörper 10 ist der Aufbewahrungs- und/oder Lagerbehälter 20, der bevorzugt aus einem Glasmaterial gefertigt und mit einem Stopfen 30, bevorzugt aus gummielastischem Werkstoff, verschlossen ist, im Wesentlichen axial beweglich in Richtung 40 eingebracht. Der Aufbewahrungs- und/oder Lagerbehälter 20 mit Verschluss 30 kann durch eine Drückeinrichtung 50 innerhalb des Spritzenkörpers axial in Richtung 40 zum vorderen Ende des Spritzenkörpers bewegt werden.

Des Weiteren umfasst die in Fig. 1 detailliert dargestellte Vorrichtung eine Nadelrückzieheinrichtung 100. Die Nadelrückzieheinrichtung wiederum weist eine Nadelhalteeinrichtung 110 sowie eine Druckfeder 120 und eine Auslöseeinrichtung 130 in Form einer Kralle auf. Die Druckfeder 120 wird durch die Federführung, die durch die Nadelhalteeinrichtung zur Verfügung gestellt wird, die optional aber nicht zwingend ist, geführt.

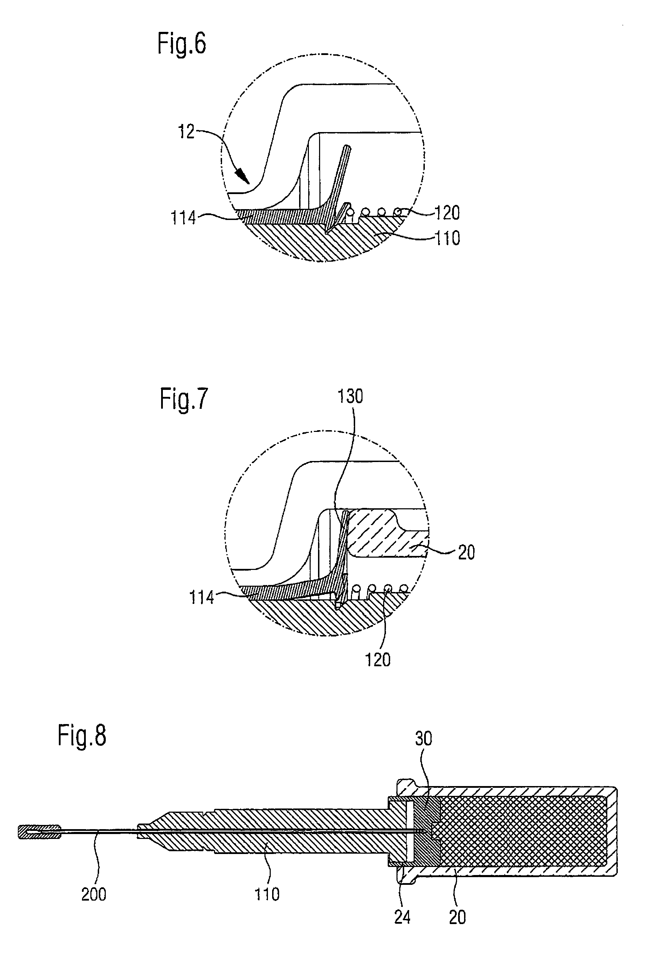

Wie in der dreidimensionalen Ansicht sind in Fig. 1 Seitenansichten der Einzelteile gezeigt. Der Spritzenkörper 10 umfasst ein vorderes Ende bzw. einen vorderen Bereich 12, in den die Nadelhalteinrichtung 110 eingesetzt werden kann, durch die die Nadel 200 hindurchgeführt wird. Des Weiteren umfasst der Spritzenkörper 10, der bevorzugt aus einem Kunststoff gefertigt ist, einen Bereich 14 mit Führungsöffnungen 16, die Vorsprünge 52 der Drückeinrichtung 50 aufnehmen können, die dazu dienen, dass die Drückeinrichtung 50 entlang der Öffnungen 16 beim axialen Verschieben in Richtung 40 geführt wird. Um die üblichen Verarbeitungsmaschinen der Vorrichtung zur Befüllung, Aufbewahrung und Lagerung von flüssigen Medien handhaben zu können, umfasst der Spritzenkörper 10 einen Spritzenkragen 18. Des Weiteren umfasst die Führungsöffnung 16 eine Ausnehmung 17, in die der Vorsprung 52 einrasten und das Innere 57 des Druckkörpers, der als Hohlkörper, hier als Hohlzylinder, ausgebildet ist, freigeben kann, so dass der Lager- und/oder Aufbewahrungsbehälter 20 beim Zurückziehen der Nadel in diesen Hohlraum verbracht werden kann, wie in Fig. 7 gezeigt.

Die Nadel, insbesondere die Spritzennadel 200 ist, wenn die Vorrichtung zur Aufbewahrung und Lagerung von flüssigen Medien gelagert wird und die gelagerte Flüssigkeit nicht ausgebracht werden soll, nicht mit dem Spritzenkörper 10 verbunden. Bevorzugt ist die Nadel, insbesondere die Spritzennadel 200 aus Edelstahl gefertigt. Die Auslöseeinrichtung 130 ist bevorzugt als Kralle bestehend bevorzugt aus Kunststoff, ausgebildet. Die Nadelhalteeinrichtung, die gleichzeitig als Federführung dient, ist mit Bezugsziffer 110 belegt und besteht bevorzugt aus Kunststoff. Die Druckfeder, die Teil der Nadelrückzieheinrichtung ist, besteht aus Federstahl. Um eine Langzeitlagerung des flüssigen Mediums, insbesondere Arzneimittel, zu ermöglichen, besteht der Vorrats- bzw. Lager- und/oder Aufbewahrungsbehälter aus einem Glasmaterial, bevorzugt in Rohrform. Als Verschlussmaterial für den rohrförmigen Glaskörper, in dem die Flüssigkeit bzw. das Arzneimittel aufgenommen wird, dient ein Verschlussstopfen 30, bevorzugt aus einem gummielastischen Material. Die Drückeinrichtung 50 zum Verschieben des Aufbewahrungs- und/oder Lagerbehälters 20, im Wesentlichen in axialer Richtung zum vorderen Ende 12 hin, ist wiederum aus Kunststoff gefertigt und weist zur Führung wenigstens einen Vorsprung 52 auf.

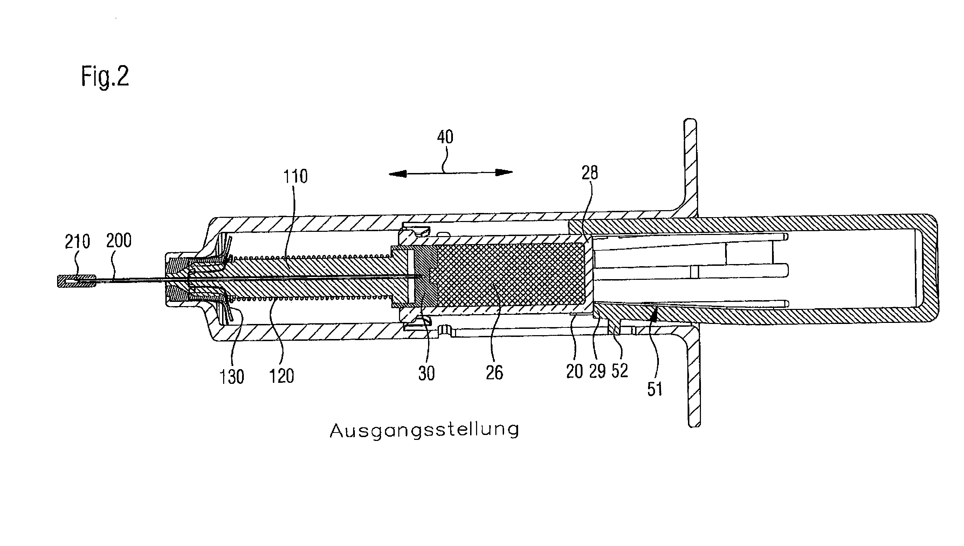

In Fig. 2 ist die erfindungsgemäße Vorrichtung zur Aufbewahrung und/oder Lagerung eines Arzneimittels in zusammengebautem Zustand in der Ausgangsstellung bei eingebrachter Nadel 200, aber vor dem Ausbringen des fluiden Mediums, insbesondere des Arzneimittels, das sich im Inneren 26 des aus Glasmaterial bestehenden Lager- und/oder Aufbewahrungsbehälters befindet, gezeigt.

Die Füllmenge des fluiden Mediums, insbesondere Arzneimittels, im Innern 26 des Aufbewahrungs- und/oder Lagerbehälters 20 beträgt, ohne hierauf beschränkt zu sein, in vorliegendem Fall beispielsweise 1 ml. Am Ende bzw. der Rückseite 28 bzw. dem Boden des Lager- und/oder Aufbewahrungsbehälters liegen die Enden 29 der Wandung, die bevorzugt Finger sind, der hohl ausgebildeten Drückeinrichtung 50 an, die über den Spritzenkragen 18 hinausragt. Wie in Fig. 2 deutlich zu erkennen ist, ist die Rückseite 28 des Lager- und Aufbewahrungsbehälters, der in Form eines Fläschchens ausgebildet ist, ein fester Boden, auf den die nachfolgend beschriebene Druckeinrichtung wirkt..

In der Ausgangsstellung in Fig. 2 ist der Vorsprung 52 der Drückeinrichtung nach unten gedrückt, so dass der vordere Abschnitt 51 der Drückeinrichtung mit dem Ende bzw. der Rückseite 28 bzw. dem Boden des Vorrats- und/oder Lagerbehälters zur Anlage kommt, so dass der Vorrats- und/oder Lagerbehälter mit Hilfe der Drückeinrichtung 50 in axialer Richtung 40 zum vorderen Ende 12 des Spritzenkörpers 10 verschoben werden kann.

Deutlich zu erkennen ist auch die in dem vorderen Teil 12 des Spritzenkörpers eingebrachte Nadelhalteeinrichtung 110, die gleichzeitig der Nadelführung sowie der Führung der Druckfeder 120 dient. Die Druckfeder 120 ist in der Ausgangsstellung in einem gespannten Zustand. Die Nadel 200 ist mit einer Kappe 210 verschlossen.

Die Nadel 200, die innerhalb der Nadelhalteeinrichtung gehalten wird, ist in den Verschluss 30 aus gummielastischem Material des Aufbewahrungs- und/oder Lagerbehälters 20 eingestochen, ohne diesen zu durchstoßen.

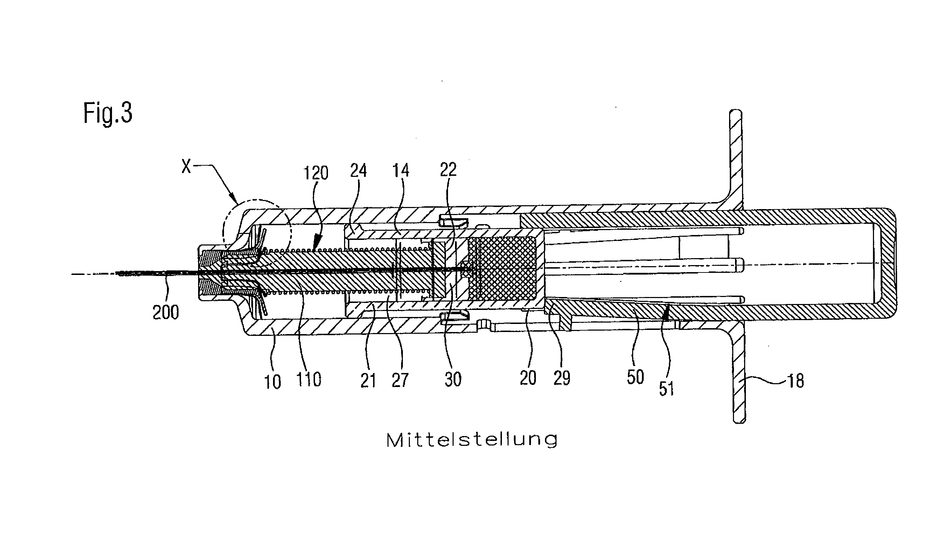

Um die im Innern 26 des Lager- und/oder Aufbewahrungsbehälters befindliche Flüssigkeit bzw. Arzneimittel auszubringen, wird mit Hilfe der Nadel 200 der Verschluss 30, wie in Fig. 3 gezeigt, durchstochen bzw. durchstoßen. Gleiche Bauteile wie in den Figuren 1 und 2 sind mit denselben Bezugsziffern bezeichnet. Durch das Durchstoßen des Verschlusses 30 kann nunmehr über die Nadel 200 die im Innern des Behälters 20 vorhandene Flüssigkeit austreten.

Durch Verschieben der niedergehaltenen Drückeinrichtung 50 in axialer Richtung 40 entlang der Führungsnut 16 des Spritzenkörpers, wie in Fig. 1 dargestellt, wird der Lager- bzw. Aufbewahrungsbehälter in Richtung des vorderen Endes 12 des Spritzenkörpers 10 bewegt und so die Flüssigkeit aus dem Innern 26 ausgebracht.

Fig. 3 zeigt in etwa die Mittenstellung des Aufbewahrungs- und/oder Lagerbehälters 20. Wie aus Fig. 3 deutlich zu erkennen, wird mit Hilfe der Drückeinrichtung 50 der hohle Aufbewahrungs- und/oder Lagerbehälter über das Verschlusselement 30 hinwegbewegt. Die Wand 21 des Aufbewahrungs- und/oder Lagerbehälters wird dabei zwischen der Innenwand 14 des Spritzenkörpers 10 sowie der Nadelhalteeinrichtung 110 mit Druckfeder 120 bewegt. Hierbei erfolgt die Führung zum einen durch Druck des Verschlusselementes 30 auf die Innenseite 22 der Wand 21 des hohlen Aufbewahrungs- und/oder Lagerbehälters sowie mit Hilfe von Führungsvorsprüngen 24 in der Wand 21 des Lager- und/oder Aufbewahrungsbehälters durch die Innenwand 14 des Spritzenkörpers 10.

Hierbei ist ein ausreichender Raum 27 zwischen der Innenwand 22 des Lager- und/oder Aufbewahrungsbehälters sowie der Druckfeder 120 vorgesehen. Gleiche Bauteile wie in den Figuren 1 bis 2 sind in Fig. 3 mit denselben Bezugsziffern versehen.

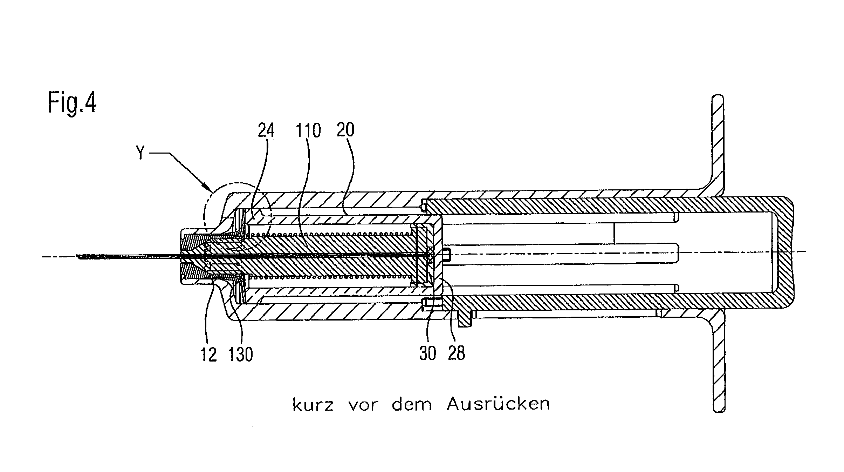

Fig. 4 zeigt die Vorrichtung vor Auslösen der Nadelrückzieheinrichtung mit Hilfe der Auslöseeinrichtung in Form einer Kralle 130. In Fig. 4 liegt das Ende bzw. die Rückwand bzw. der Boden 28 des Glasbehälters am Verschluss 30 an und bis auf eine Restmenge R ist der vollständige Inhalt des Aufbewahrungs- und/oder Vorratsbehälters aus dem Inneren 26 durch die Nadel 200 ausgebracht.

Das Auslösen der Nadelrückzieheinrichtung mit Hilfe der Auslöseeinrichtung ist in Fig. 4 detailliert dargestellt. Hierzu wird mit Hilfe der Drückeinrichtung 50 der Verschluss 30 aus einem gummielastischen Werkstoff komprimiert. Hierdurch wird zum einen die noch verbliebene Restmenge R des fluiden Mediums, insbesondere Arzneimittels, weiter reduziert, zum anderen wird durch die Vorsprünge 24 des Aufbewahrungs- und/oder Lagerbehälters 20 die Kralle 130 aufgeweitet. Der Formschluss der Nadelhalteeinrichtung 110 mit der Kralle, die mit dem vorderen Ende 12 des Spritzenkörpers 10 fest verbunden ist, wird aufgehoben. Durch den Druck des Aufbewahrungs- und/oder Lagerbehälters auf die Kralle 130 wird diese aufgeweitet und die Nadelhalteeinrichtung wird ausgelöst bzw. losgelassen.

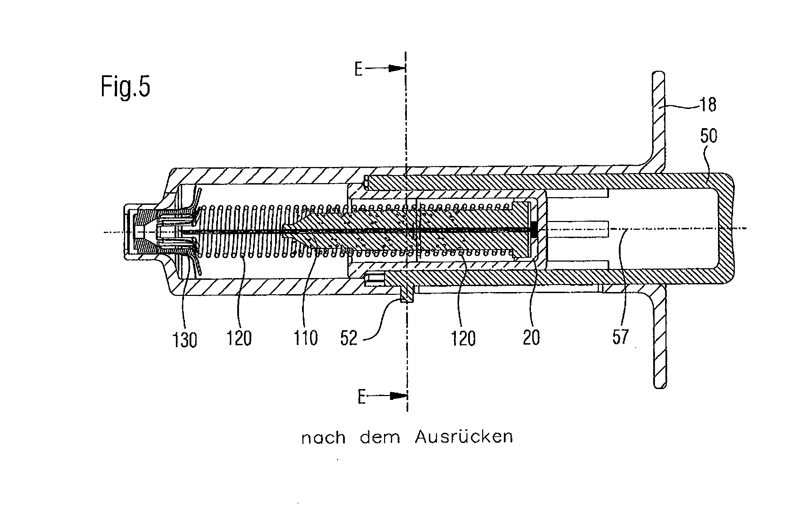

Durch das Aufheben des Form- bzw. Reibschlusses zwischen der Nadelhalteeinrichtung 110 und dem vorderen Ende 12 des Spritzenkörpers kann sich die Druckfeder 120 entspannen, so dass, wie in Fig. 5 gezeigt, sich die Nadelhalteeinrichtung 110, die mit der Nadel 200 fest bzw. unlösbar, z. B. geklebt oder verpresst, verbunden ist, in axialer Richtung in Richtung des Spritzenkragens 18 bewegt.

Zusammen mit der Nadelhalteeinrichtung 110 bewegt sich der Lager- und/oder Aufbewahrungsbehälter 20 in das Innere 57 der hohlen Drückeinrichtung 50 zurück. Dies ist möglich, da das Innere der Drückeinrichtung durch Verrasten des Kragens 52 der Drückeinrichtung in der Ausnehmung 17 des Spritzenkörpers freigegeben wird zur Aufnahme des Aufbewahrungs- und/oder Lagerbehälters 20.

Das Zurückziehen der Nadel sowie des Lager- und/oder Aufbewahrungsbehälters 20 erfolgt durch Entspannen der Druckfeder und die bei der Entspannung auf den Verschluss 30 wirkende Kraft.

Gleiche Bauteile wie in den Figuren 1 bis 4 werden in Fig. 5 mit denselben Bezugsziffern versehen.

In den Figuren 6 bis 7 ist detailliert das Auslösen der Nadelrückzieheinrichtung dargestellt. Deutlich zu erkennen ist die Druckfeder 120, die die Nadelhalteeinrichtung 110 umgibt. Die Nadelhalteeinrichtung wird durch einen Formschluss im Bereich des Vorsprungs 114 im vorderen Bereich des Spritzenkörpers gehalten. Der Einsatz mit der Kralle 130, der die formschlüssige Verbindung zu dem Nadelhaltekörper darstellt, ist mit dem vorderen Bereich des Spritzenkörpers fest verbunden, beispielsweise durch Kleben. Hierdurch wird der Nadelhaltekörper bzw. die Nadelhalteeinrichtung im vorderen Ende 12 des Spritzenkörpers 10 gehalten.

Der Lager- und/oder Aufbewahrungsbehälter ist in Fig. 7 mit 20 bezeichnet. Wird, wie in Fig. 7, der Lager- bzw. Aufbewahrungsbehälter 20 gegen die Kralle 130 gedrückt, so wird die Kralle aufgedehnt und der Formschluss im Bereich 114 mit dem Nadelhaltekörper 110 aufgehoben. Der Nadelhaltekörper ist somit freigegeben und kann sich, wie in Fig. 5 gezeigt, durch Entspannen der Feder 120 in Richtung des Spritzeninneren.

In Fig. 8 sind die Bauteile der Vorrichtung dargestellt, die sterilisiert werden und gemeinsam, als sterile Einheit, mit den restlichen, nicht sterilen Komponenten montiert bzw. zusammengebaut werden. Die Nadel wird durch die Kappe und den angestochenen Verschluss bis zur Verabreichung steril gehalten. Lediglich der Verschluss und der Aufbewahrungsbehälter stehen mit dem Arzneimittel bis zur Verabreichung in Kontakt. Gleiche Bauteile wie in den vorangegangenen Figuren sind mit denselben Bezugsziffern gekennzeichnet. Hierbei handelt es sich um die Nadel 200, den Gummistopfen 30, den Lager- und/oder Aufbewahrungsbehälter 20 sowie die Nadelhalteeinrichtung 110.

Mit der Erfindung wird somit erstmals eine Vorrichtung zur Aufbewahrung und/oder Lagerung von flüssigen Medien, insbesondere Arzneimitteln, angegeben, die mit einer Nadel ausgebracht werden können und über eine Nadelrückzieheinrichtung verfügen, wobei das Medikament bei der Lagerung nur mit Glas und Gummi Kontakt hat und kein Umfüllen oder weiterer Montageschritt notwendig ist.

Die Erfindung umfasst Aspekte, die in nachfolgenden Sätzen offenbart sind, die Teil der Beschreibung sind, aber keine Ansprüche gemäß J 15/88

### Sätze

1. Vorrichtung zur Aufbewahrung und Lagerung von flüssigen Medien, insbesondere Arzneimitteln mit einem Spritzenkörper (10), umfassend einen Aufbewahrungs- und/oder Lagerbehälter (20), insbesondere aus einem Glasmaterial oder einem Cyclooelefincopolymer (COC, COP) für flüssige Medien, insbesondere Arzneimittel, mit einem Verschluss (30), insbesondere aus einem gummielastischen Werkstoff sowie wenigstens einer Nadel (200) und
   einer Nadelrückzieheinrichtung (100),
   dadurch gekennzeichnet, dass
   der Aufbewahrungs- und/oder Lagerbehälter als Fläschchen mit einem festen Boden (28) und die Vorrichtung eine Schiebe-, insbesondere eine Drückeinrichtung (50) umfasst zur Verschiebung des Aufbewahrungs- und/oder Lagerbehälters (20) im Wesentlichen in axialer Richtung (40) in dem Spritzenkörper (10), wobei die Schiebe-, insbesondere Drückeinrichtung auf den festen Boden (28) wirkt.
2. Vorrichtung nach Satz 1,
   dadurch gekennzeichnet, dass
   die Drückeinrichtung wenigstens einen Vorsprung (52) umfasst.
3. Vorrichtung nach Satz 2,
   dadurch gekennzeichnet, dass
   die Drückeinrichtung einen Hohlkörper, insbesondere einen Hohlzylinder mit wenigstens einer Öffnung umfasst.
4. Vorrichtung nach einem der Sätze 2 bis 3,
   dadurch gekennzeichnet, dass
   der Vorsprung (52) derart ausgebildet ist, dass die Drückeinrichtung (50) zum Verschieben des Aufbewahrungs- und/oder Lagerbehälters (20) zum Ausbringen des flüssigen Mediums mit dem Aufbewahrungs- und/oder Lagerbehälter (20) im Bereich des Bodens des Aufbewahrungs- und/oder Lagerbehälters zur Anlage kommt
5. Vorrichtung nach einem der Sätze 2 bis 4,
   dadurch gekennzeichnet, dass
   der Vorsprung (52) derart ausgebildet ist, dass nach Ausbringen des flüssigen Mediums aus dem Aufbewahrungs- und/oder Lagerbehälter (20) der Aufbewahrungs- und/oder Lagerbehälter von der Drückeinrichtung (50), insbesondere dem Hohlkörper, bevorzugt dem Hohlzylinder, aufgenommen wird.
6. Vorrichtung nach einem der Sätze 1 bis 5,
   dadurch gekennzeichnet, dass
   der Spritzenkörper (10) ein vorderes Ende (12) umfasst und eine Nadelhalteeinrichtung (110), die Teil der Nadelrückzieheinrichtung (100) ist und in dem vorderen Ende (12) gehalten wird.
7. Vorrichtung nach einem der Sätze 1 bis 6,
   dadurch gekennzeichnet, dass
   die Vorrichtung eine Auslösevorrichtung (130) für die Nadelrückzieheinrichtung (100) umfasst, bevorzugt eine Kralle (130).
8. Vorrichtung nach einem der Sätze 1 bis 7,
   dadurch gekennzeichnet, dass
   die Nadelrückzieheinrichtung (100) die Nadelhalteeinrichtung (110) und eine Druckfeder (120) umfasst.
9. Verfahren zum Ausbringen von flüssigen Medien, insbesondere Arzneimitteln aus einer Vorrichtung zur Aufbewahrung und/oder Lagerung gemäß einem der Ansprüche 1 bis 8, umfassend folgende Schritte:
   - der Aufbewahrungs- und/oder Lagerbehälter (20) in Form eines Fläschchens mit einem festen Boden (28)wird durch einen Einstich einer Nadel (200) in einen Verschluss (30) geöffnet;
   - das flüssige Medium, insbesondere Arzneimittel, wird durch axiales Verschieben des Aufbewahrungs- und/oder Lagerbehälters (20) in axialer Richtung (40) in dem Spritzenkörper (10) mit Hilfe einer Drückeinrichtung (50), die auf den festen Boden (28) wirkt, durch die Nadel (200) ausgebracht;
   - nach weitgehend vollständigem Ausbringen des flüssigen Mediums, insbesondere Arzneimittels, wird die Nadelrückzieheinrichtung (100) freigegeben, derart, dass die Nadel (200) in den Spritzenkörper (10) weitgehend vollständig eingezogen wird und
   - der Aufbewahrungs- und/oder Lagerbehälter (20) wird in die Drückeinrichtung (50) zurückgezogen.
10. Verfahren nach Satz 9,
   dadurch gekennzeichnet, dass
   durch eine Auslöseeinrichtung (130) der Halt der Nadelrückzieheinrichtung (100) im vorderen Ende (12) des Spritzenkörpers (10) geöffnet wird, so dass bevorzugt eine Druckfeder (120) sich entspannt und dadurch eine Nadelhalteeinrichtung (110) mit der Nadel (200) in das Innere des Spritzenkörpers (10) gezogen wird.
11. Verfahren nach einem der Sätze 9 bis 10,
   dadurch gekennzeichnet, dass
   durch die Auslöseeinrichtung (130) ein Form- und/oder Reibschluss der Nadelhalteeinrichtung (110) aufgehoben und dadurch die Nadelrückzieheinrichtung (100) freigegeben wird.

## Patentansprüche

1. Vorrichtung zur Aufbewahrung und Lagerung von flüssigen Medien, insbesondere Arzneimitteln mit einem Spritzenkörper (10), umfassend einen Aufbewahrungs- und/oder Lagerbehälter (20), insbesondere aus einem Glasmaterial oder einem Cyclooelefincopolymer (COC) oder einem Cyclooelefinpolymer (COP) für flüssige Medien, insbesondere Arzneimittel, mit einem Verschluss (30), insbesondere aus einem gummielastischen Werkstoff sowie wenigstens einer Nadel (200) und
einer Nadelrückzieheinrichtung (100),
**dadurch gekennzeichnet, dass**
der Aufbewahrungs- und/oder Lagerbehälter mit einem festen Boden (28), insbesondere als Fläschchen ausgebildet ist und die Vorrichtung eine Schiebe-, insbesondere eine Drückeinrichtung (50) umfasst zur Verschiebung des Aufbewahrungs- und/oder Lagerbehälters (20) im Wesentlichen in axialer Richtung (40) in dem Spritzenkörper (10), wobei die Schiebe-, insbesondere Drückeinrichtung auf den festen Boden (28) wirkt.

2. Vorrichtung nach Anspruch 1,
**dadurch gekennzeichnet, dass**
die Drückeinrichtung wenigstens einen Vorsprung (52) umfasst.

3. Vorrichtung nach Anspruch 2,
**dadurch gekennzeichnet, dass**
die Drückeinrichtung einen Hohlkörper, insbesondere einen Hohlzylinder mit wenigstens einer Öffnung umfasst.

4. Vorrichtung nach einem der Ansprüche 2 bis 3,
**dadurch gekennzeichnet, dass**
der Vorsprung (52) derart ausgebildet ist, dass die Drückeinrichtung (50) zum Verschieben des Aufbewahrungs- und/oder Lagerbehälters (20) zum Ausbringen des flüssigen Mediums mit dem Aufbewahrungs- und/oder Lagerbehälter (20) im Bereich des Bodens des Aufbewahrungs- und/oder Lagerbehälters zur Anlage kommt

5. Vorrichtung nach einem der Ansprüche 2 bis 4,
**dadurch gekennzeichnet, dass**
der Vorsprung (52) derart ausgebildet ist, dass nach Ausbringen des flüssigen Mediums aus dem Aufbewahrungs- und/oder Lagerbehälter (20) der Aufbewahrungs- und/oder Lagerbehälter von der Drückeinrichtung (50), insbesondere dem Hohlkörper, bevorzugt dem Hohlzylinder, aufgenommen wird.

6. Vorrichtung nach einem der Ansprüche 1 bis 5,
**dadurch gekennzeichnet, dass**
der Spritzenkörper (10) ein vorderes Ende (12) umfasst und eine Nadelhalteeinrichtung (110), die Teil der Nadelrückzieheinrichtung (100) ist und in dem vorderen Ende (12) gehalten wird.

7. Vorrichtung nach einem der Ansprüche 1 bis 6,
**dadurch gekennzeichnet, dass**
die Vorrichtung eine Auslösevorrichtung (130) für die Nadelrückzieheinrichtung (100) umfasst, bevorzugt eine Kralle (130).

8. Vorrichtung nach einem der Ansprüche 1 bis 7,
**dadurch gekennzeichnet, dass**
die Nadelrückzieheinrichtung (100) die Nadelhalteeinrichtung (110) und eine Druckfeder (120) umfasst.

9. Vorrichtung nach einem der Ansprüche 1 bis 8,
**dadurch gekennzeichnet, dass**
der Aufbewahrungs- und/oder Lagerbehälter (20) aus einem Glasmaterial oder einem Cyclooelefincopolymer (COC) oder einem Cyclooelefinpolymer (COP) besteht.

10. Vorrichtung nach einem der Ansprüche 1 bis 9,
**dadurch gekennzeichnet, dass**
der Verschluss aus einem gummielastischen Werkstoff besteht.

11. Verfahren zum Ausbringen von flüssigen Medien, insbesondere Arzneimitteln aus einer Vorrichtung zur Aufbewahrung und/oder Lagerung gemäß einem der Ansprüche 1 bis 9, umfassend folgende Schritte:
- der Aufbewahrungs- und/oder Lagerbehälter (20) mit einem festen Boden (28), insbesondere in Form eines Fläschchens wird durch einen Einstich einer Nadel (200) in einen Verschluss (30) geöffnet;
- das flüssige Medium, insbesondere Arzneimittel, wird durch axiales Verschieben des Aufbewahrungs- und/oder Lagerbehälters (20) in axialer Richtung (40) in dem Spritzenkörper (10) mit Hilfe einer Drückeinrichtung (50), die auf den festen Boden (28) wirkt, durch die Nadel (200) ausgebracht;
- nach weitgehend vollständigem Ausbringen des flüssigen Mediums, insbesondere Arzneimittels, wird die Nadelrückzieheinrichtung (100) freigegeben, derart, dass die Nadel (200) in den Spritzenkörper (10) weitgehend vollständig eingezogen wird und
- der Aufbewahrungs- und/oder Lagerbehälter (20) wird in die Drückeinrichtung (50) zurückgezogen.

12. Verfahren nach Anspruch 11,
**dadurch gekennzeichnet, dass**
durch eine Auslöseeinrichtung (130) der Halt der Nadelrückzieheinrichtung (100) im vorderen Ende (12) des Spritzenkörpers (10) geöffnet wird, so dass bevorzugt eine Druckfeder (120) sich entspannt und dadurch eine Nadelhalteeinrichtung (110) mit der Nadel (200) in das Innere des Spritzenkörpers (10) gezogen wird.

13. Verfahren nach einem der Ansprüche 11 bis 12,
**dadurch gekennzeichnet, dass**
durch die Auslöseeinrichtung (130) ein Form- und/oder Reibschluss der Nadelhalteeinrichtung (110) aufgehoben und dadurch die Nadelrückzieheinrichtung (100) freigegeben wird.
